Europäisches Patentamt

European Patent Office

Office européen des brevets

(11) Publication number: **0 310 227**
**A1**

# EUROPEAN PATENT APPLICATION

(21) Application number: 88307019.5

(51) Int. Cl.⁴: **A61M 5/28**

(22) Date of filing: 29.07.88

The title of the invention has been amended (Guidelines for Examination in the EPO, A-III, 7.3).

(30) Priority: 30.07.87 GB 8718116

(43) Date of publication of application:
05.04.89 Bulletin 89/14

(84) Designated Contracting States:
AT BE CH DE ES FR GB GR IT LI LU NL SE

(71) Applicant: WAVERLEY PHARMACEUTICAL
LIMITED
Goddard Road Astmoor
Runcorn Cheshire WA7 1QE(GB)

(72) Inventor: Rose, Howard
55A Popes Avenue Strawberry Hill
Twickenham Middlesex TW2 5TN(GB)
Inventor: McAffer, Ian Gardner Cameron
88 Mount Pleasant Biggin Hill
Westerham Kent TN16 3NF(GB)

(74) Representative: Ruffles, Graham Keith et al
MARKS & CLERK 57-60 Lincoln's Inn Fields
London WC2A 3LS(GB)

(54) Injection device.

(57) The present invention relates to injection device comprising a squeezable, moulded plastics ampoule (10) with a male head (16) together with a hollow, open-ended needle (18) having a co-operating female recess (20), the whole providing rapid, one-off, disposable injection devices.

FIG.3.

## Injection Systems

The present invention relates to injection systems and ampoules for use therein.

Injection systems for liquids and gases come in many shapes and forms, common amongst which are syringes. The dispensing member of the syringe is the needle, which is often detachable/disposable, especially where medical use is envisaged. Syringe bodies are generally considerably more expensive to produce than the needles, and so it is common to re-use the barrel, as its sterility need not be compromised by patient contact.

Syringes are well known which incorporate a luer fitting for connection of the syringe barrel to a needle. Essentially the luer fitting utilises a tapered male luer head on the syringe barrel and a correspondingly tapered female luer recess at the rear of the needle.

However, in order to use the above system, it is first necessary to fill the barrel of the syringe. This can either be effected by drawing up the liquid prior to fitting the needle or after, both methods being time-consuming, the second also being dangerous to the user when a surface must be punctured to gain access to the liquid.

In accordance with the present invention there is provided an injection system comprising a squeezable moulded plastics ampoule having a male head together with a hollow, open-ended needle having a co-operating female recess.

The preferred fitting between ampoule and needle is the luer, which comprises a male cone, with a conicity of about 6:100, co-operating with a corresponding inverted female cone. However it is equally feasible to use other conical forms, such as, for example, the recordal system, which has a conicity of about 1:10. In general, the greater the divergence from parallel, the less secure is the fit. In fact, especially when using such materials as high density polypropylene, even parallel sides providing a cylindrical fit can provide a sufficiently snug fit for the purposes of the invention, and even a slightly everted cone, providing a snap-fit, may be used, although this may lead to difficulties in practice.

The fit between ampoule and needle may also comprise a screw-thread and/or a retaining collar. A screw-thread is not generally necessary, a push fit being preferable. The collar is conveniently provided on the ampoule and may either protrude, or take the form of a recess in one end of the ampoule with the male head located inside.

The ampoule is preferably of the kind which is manufactured, filled and sealed in a mould. Any convenient shape or form may be used, but it is generally preferred for ampoules to be generally cylindrical, or slightly flattened cylindrical. Suitable plastics materials for the ampoule include polyethylene, polypropylene and polyvinyl chloride.

The ampoule should be squeezable when open to enable expulsion of the contents. The degree of squeezability depends on the use for which the system is intended. A very soft ampoule, collapsing on punctured, may be of use in certain medical or rapid-use situations, while a virtually solid ampoule may be required where mechanical force is exerted to expel the contacts.

Preferably, the ampoule contains a unit dose of the relevant substance, such as a medicine. In certain circumstances, it may be appropriate to provide a multiple or fraction of the unit dose. It may also be convenient to provide a slight excess to account for any residue left in the neck. In an alternative, air or an inert gas may be incorporated such that, when the injection system is used needle-down, the air bubble is expelled last, ensuring complete ejection of the liquid. Such a system may be inappropriate in certain cases, such as for injection direct into the blood-stream.

The ampoule may contain any suitable substance susceptible of use with the system. The substance may be solids, liquid or gas. In the case of non-flowing solids, it may be necessary to warm or otherwise treat the ampoule to achieve the desired state prior to use. Liquids can include colloids, suspensions, solutions and suitable mixtures, while gases may be chosen as appropriate, and may include bromine, for example, or other gases required in a pure state.

The needle is conveniently shaped to provide a sharp, protruding edge to facilitate injection and to lessen the chances of taking a core of, for example, flesh. Nevertheless, certain systems may not require such a refinement, and a straw-like needle may suffice.

In order to avoid unnecessary exposure of its contents, the ampoule is preferably sealed. The form the seal may take is largely dictated by convenience, but may for example, take the form of a direct or torque-action, snap-off cap, or a membrane intended to break in use. In the latter case, the membrane may be designed to rupture only when sufficient force is exerted on the ampoule, or when the head of the ampoule is fitted into the needle recess. Rupture on fitting in the recess is conveniently achieved by a portion of the needle extending back into the recess, although other suitable means may also be employed.

The injection systems of the invention may be provided as their separate components or together in the same package. In one embodiments, the ampoule and needle are pre-engaged, only a final push being necessary to puncture a sealing membrane, or a squeeze to rupture the membrane.

The injection system of this invention can be used instead of a syringe for various uses, including injection in to a human or animal, or injection in to a delivery system, such as a drip bottle, or delivery into an experimental system in the laboratory, for example.

It will be appreciated that the injection systems of the present invention may be used independently, or as part of a larger system. For example, an injection gun may be fitted with a permanent or replaceable needle, and ampoules may be fitted one at a times or provided in a cartridge, for dispensation. In such a gun, it is convenient to crush the ampoule by a suitable mechanism, such as by jaws or by a roller or cam action, and which may be operated mechanically or by manual force through a trigger, for example.

It will be appreciated that a great advantage of the present invention lies in the convenience of the ampoules to provide a one-off, disposable system, avoiding the need for sterilisation, accurate measurement and filling and unnecessary exposure to needles, especially in the age of AIDS. The system is fast, accurate and easy to use, and possesses great flexibility.

The present invention will now be further illustrated with reference to the accompanying drawings in which:

Figure 1 shows an ampoule and needle in accordance with the invention;

Figure 2 shows an alternative ampoule and needle in accordance with the invention; and

Figure 3 shows a complete system in accordance with the invention.

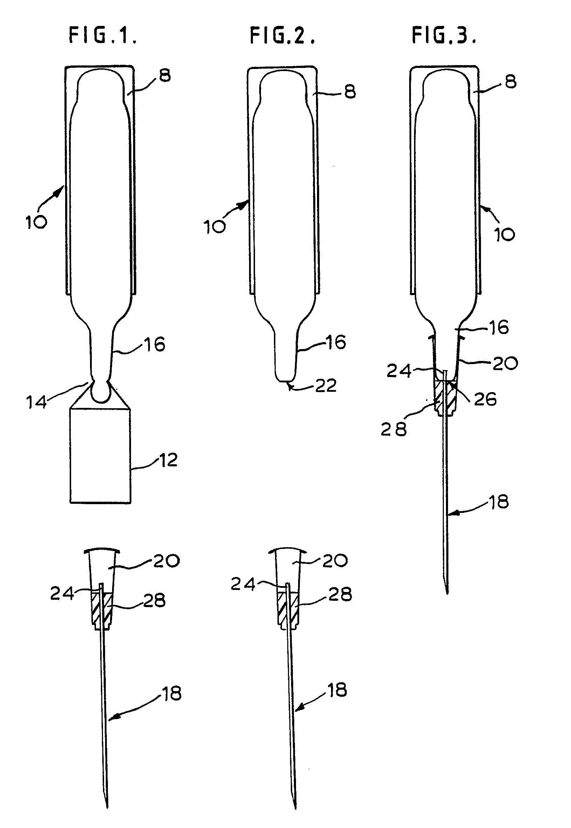

In Figure 1, an ampoule 10, moulded, for example, from polyethylene, has a tear-off tab 12 connected by a rupturable neck 14 to a head 16 of the ampoule. In a known manner, the ampoules are manufactured, filled and sealed in a mould.

In accordance with a preferred embodiment of this invention, the head 16 of the ampoule 10 is a male luer head. Specifically, the head 16 takes a hollow frustoconical form, with a reducing taper towards the outer end. Although not clearly shown in the drawing, the angle of taper is that required for a luer fitting. A flange 8 is provided about the ampoule 10 to facilitate gripping, fitting into a device or automated manufacture.

For use with the ampoule 10 there is provided a needle 18 with a female luer recess 20. The female luer 20 is shown with a rearwardly projecting posterior end 24 of the needle, adapted to mate with the delivery channel in the ampoule head 16. It is not necessary to have inward extension 24 projecting within the female luer, and the end of the needle 24 may instead be located within, or flush with, the solid retaining part 28 of the female luer.

In use, the tab 12 is used to open the ampoule and the luer head 16 is then mated to the needle 18. The contents are then delivered by squeezing the ampoule 10.

Alternatively, the needle can form part of a gun, with a roller or other mechanism adapted to travel and express fluid from the ampoule through a needle.

In Figure 2, an alternative ampoule is shown which has a rupturable membrane 22 sealing the open end of head 16. When ampoule 10 and needle 18 are mated, the posterior end 24 of the needle pierces the membrane 22 to allow expression of the ampoule's contents.

Figure 3 shows a mated ampoule 10 and needle 18 forming an injection system in accordance with the invention. The open end 26 of the head 16 seats over the posterior 24 of the needle which further helps to seal the system by contact of the needle with the interior walls of head 16. The exterior walls of head 16 contact the interior walls of female luer 20 completing an air-tight seal.

## Claims

1. An injection system comprising a squeezable moulded plastics ampoule having a male head together with a hollow, open-ended needle having a co-operating female recess.

2. An injection system according to claim 1 wherein the ampoule is filled with an injectable substance.

3. An injection system according to claim 1 or 2 wherein the male head and female recess are co-operating male and female cones.

4. An injection system according to claim 3 wherein the male head and female recess are co-operating luer fittings.

5. An injection system according to any preceding claim wherein the female recess is provided in a plastics body bonded about the base of the needle.

6. An injection system according to claim 5 wherein the base of the needle protrudes into the female recess.

7. An injection system according to any preceding claim wherein the base of the needle is adapted to enter the male head on the mating of ampoule and needle.

8. An injection system according to any preceding claim wherein the head of the ampoule is sealed by a snap-off cap.

9. An injection system according to any of claims 1 to 7 wherein the head of the ampoule is sealed by a rupturable membrane.

10. An ampoule for use with a system as described in any preceding claim.

FIG.1.

FIG.2.

FIG.3.

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| X | US-A-2 687 727 (LAWSHE)<br>* Description; figures * | 1-7,9, 10 | A 61 M 5/28 |
| X | US-A-2 911 972 (ELINGER)<br>* Description; figures * | 1-8,10 | |
| | | | **TECHNICAL FIELDS SEARCHED (Int. Cl.4)**<br><br>A 61 M |

The present search report has been drawn up for all claims

| Place of search | Date of completion of the search | Examiner |
|---|---|---|
| THE HAGUE | 07-11-1988 | VANRUNXT J.M.A. |